# EUROPEAN PATENT SPECIFICATION

(11) **EP 4 037 724 B1**
(45) Date of publication and mention of the grant of the patent: **28.01.2026**
(21) Application number: 20872488.0
(22) Date of filing: 24.09.2020
(51) Int. Cl.: A61M 1/02

(54) **BLOOD DONATION SYSTEM**
BLUTSPENDESYSTEM
SYSTÈME DE DON DE SANG

(30) Priority: 30.09.2019 US 201962907843 P
(43) Date of publication of application: 10.08.2022
(73) Proprietor: Fresenius Kabi (Guangzhou) Co., Ltd., Guangzhou, Guangdong 510730 (CN)
(72) Inventor: PLUSKWIK, Joerg, Guangzhou, Guangdong 510730 (CN)
(74) Representative: Maikowski & Ninnemann Patentanwälte Partnerschaft mbB
(86) International application number: PCT/CN2020/117282
(87) International publication number: WO 2021/063234

(56) References cited:
- CA-A1- 2 386 040
- CN-A- 102 430 161
- CN-A- 102 430 161
- CN-A- 104 248 511
- CN-A- 106 384 322
- CN-A- 107 551 338
- CN-U- 202 342 048
- CN-U- 204 582 095
- CN-Y- 2 681 749
- CN-Y- 201 418 869
- FR-A1- 2 976 667
- US-A1- 2006 180 526

## Description

### FIELD

The administration of blood or blood components often plays a critical role in the emergency and/or long term treatment of patients. Blood or the individual components of blood (such as platelets, plasma, red blood cells, etc.) may be administered or transfused to patients to treat a variety of conditions. For example, blood may be administered to a patient to replace blood lost as a result of trauma, while individual blood components may be administered as part of a longer term treatment of patients suffering from cancer or certain blood related diseases. The blood or blood components administered to the patient come from blood previously collected from donors. A prior art device is disclosed e.g. in CA 2386040 A1.

### BACKGROUND

One of the most common blood collection techniques is the manual collection of whole blood from healthy donors. As commonly understood and as used herein, "manual" collection refers to a collection method where whole blood is allowed to drain from the donor and into a collection container without the use of external pumps or similar devices. This is in contrast to the so-called "automated" procedures where blood is withdrawn from a donor and further processed by an instrument that typically includes a processing or separation device and pumps for moving blood or blood components into and out of the device.

Manual collection typically utilizes a blood donation system ("BDS") that includes a venous access device and one or more collection containers interconnected by tubings. Withdrawing blood from the donor includes inserting a venous access device, such as a needle, into the donor's arm, and withdrawing blood from the donor through the needle. A plastic tube connected to the venous access device provides a flow path for the blood to the one or more pre-attached plastic blood containers or bags for collecting the blood. The BDS is pre-sterilized and disposed of after a single use.

In the manual technique, the collection container and plastic tubing may also include a volume of a liquid anticoagulant. Anticoagulant is required because of the tendency of blood to clot and adhere to the walls of the plastic surfaces which it contacts.

A serologic/pathologic assay is typically performed on blood samples taken at the beginning of the blood collection by means of a sampling system that forms part of the blood collection system. See, e.g., US 6,387,086. After collection and processing, there may be need for further samples of the blood or its components (such as red cell concentrate ("RCC"), plasma or platelets) for cross matching the donated blood component with the patient's blood prior to infusion. For this purpose, it is known to create separate samples in sealed segments of the tubing that comprise the blood collection system. That is, a tubing segment which contains a volume of whole blood or blood component is sealed on either end (with a heat sealer).

Each tubing segment has an alphanumeric identification code applied to it that permits the traceability of blood and its components to a specific donation, with each tube segment of a single donation having the same unique alphanumeric number. Thus, the sample in a tube segment can be related to a collection container having an identical identification code associated with it to insure that the blood product that is to be reinfused is the same as the sample that was cross matched to the patient. However, the alphanumeric identification code applied to the tubing segment can be difficult to read. As the alphanumeric identifier is typically read by a human operator, this introduces the potential element of human error.

By way of the present disclosure, a blood donation system is provided that provides for more accurate and reliable reading of the identification code associated with the sample segments and facilitates the automation of record keeping with respect to the donation.

### SUMMARY

The invention is defined by the appended claims. The blood donation system described herein has several aspects. In a first aspect, a blood donation system is provided comprising a donor access device; a first collection container having an inlet port and an outlet port; a first tubing connecting the donor access device to the inlet port of the first collection container; a leukocyte reduction filter having an inlet and an outlet; a second collection container having an inlet port and an outlet port; a second tubing connecting the outlet port of the first collection container to the inlet of the leukocyte reduction filter; and a third tubing connecting the outlet of the leukocyte reduction filter to the inlet port of the second collection container. The system further has a machine-readable identification code associated therewith and the machine-readable identification code is printed on one of the tubings at repeated intervals to define a plurality of discrete segments, each sized to contain a sample volume of biological fluid.

In a second aspect, the machine-readable identification code of the blood donation system is printed on the third tubing segment.

According to the invention as claimed, the machine-readable identification code of the blood donation system is a barcode.

According to the invention as claimed, the barcode is a negative barcode.

In a fifth aspect, the first collection container of the blood donation system is prefilled with a volume of anticoagulant.

In a sixth aspect, an alphanumeric equivalent of the machine-readable identification code is printed on each discrete segment of the tubing in conjunction with the machine-readable identification code.

In a seventh aspect, a blood donation system is provided comprising a plurality of containers interconnected by a plurality of tubing segments, at least one tubing segment having a machine-readable identification code associated therewith printed on the tubing segment at repeated intervals to define a plurality of discrete sub-segments, each sized to contain a sample volume of biological fluid.

According to the invention as claimed, the machine-readable identification code is a barcode.

According to the invention as claimed, the barcode is a negative barcode.

In a tenth aspect, an alphanumeric equivalent of the machine-readable identification code is printed on each discrete sub-segment of the tubing segment in conjunction with the machine-readable identification code.

### BRIEF DESCRIPTION OF DRAWINGS

FIG. 1 is a perspective view of a disposable blood donation system of the type that may include an identification code in accordance with the present disclosure.

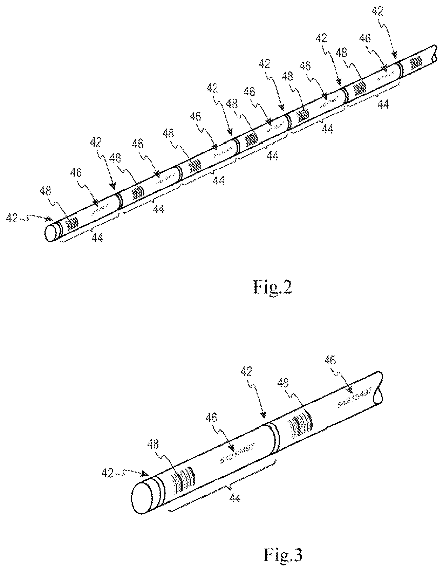

FIGS. 2 and 3 are enlarged perspective views of a tubing segment of the blood donation system of Fig. 1 in which the tubing segment includes an identification code in accordance with the present disclosure.

### DETAILED DESCRIPTION

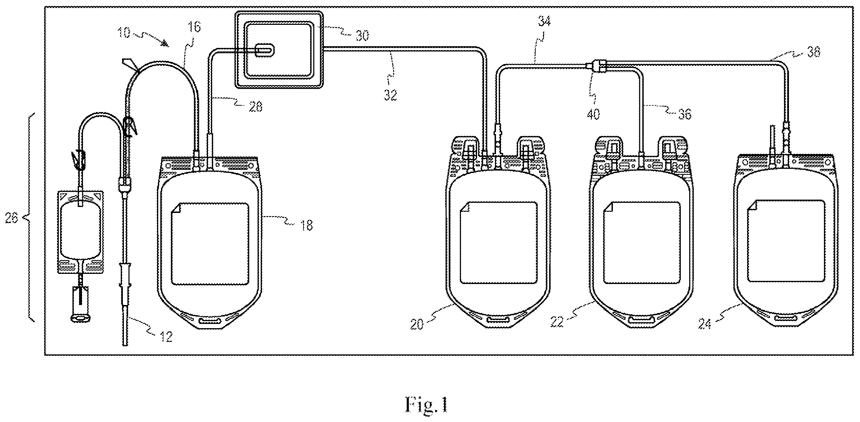

Turning now to FIG. 1 of the drawings, the present invention may be embodied in a liquid flow conduit set such as a disposable processing set 10, which is particularly suitable for use in the manual collection of blood from a donor. The illustrated disposable set 10 may include a needle such as venipuncture needle 12, and plastic tubings 14 and 16 extending from needle 12 to the inlet port of a collection container 18. A needle protector (not shown) may also be provided for retraction and storage of needle 12 after use.

The blood processing set 10 may include a single blood collection container 18 or, more preferably, as shown in FIG. 1, may be a multiple blood container system including additional containers 20, 22 and 24. Containers 16, 20, 22 and 24 and associated tubing segments of processing set 10 are typically made from conventional and approved medical grade plastic materials. One such material may be polyvinyl chloride that includes a plasticizer such as, but not limited to, plasticizers selected from the family of citrate esters, which are described in U.S. Pat. Nos. 5,167,657, 5,100,401 and 5,026,347. Alternatively, and depending in part on the blood components to be stored, containers may be made from other materials such as polyolefin materials with or without plasticizer.

As illustrated, disposable processing set 10 includes a sampling system 26 for pre-donation use or for use at the beginning of collection of blood from the donor. Further details as to the sampling system may be found in, e.g., US 6,387,086, referenced above.

As set forth above, blood processing set 10 includes a primary container 18 and one or more integrally attached transfer containers 20 and 22 and container 24, which in the context of the disclosed system contains a preservative/additive solution. During use, primary container 18 (sometimes referred to as the donor bag) receives whole blood from the donor through integrally attached donor tubings 14 and 16 and venipuncture needle 12. Container 18 typically includes a suitable anticoagulant, such as citrate phosphate dextrose (CPD), citrate phosphate dextrose adenine (CPDA) or acid citrate dextrose (ACD).

As Illustrated, a tubing 28 connects the outlet port of primary container 18 to the inlet of a leukocyte reduction filter 30, while tubing 32 connects the outlet of the leukocyte reduction filter 30 to the inlet port of transfer container 20. The outlet port of transfer container 20 may be attached to the single port of transfer container 22 by tubings 34 and 36, and to the single port of container 24 by tubings 34 and 38, the tubings 36 and 38 being attached to tubing 34 by a Y connector 40.

Transfer containers 20 and 22 are provided to receive blood components such as, but not limited to, leukocyte-reduced whole blood, red blood cells and plasma that have been separated from whole blood. For example, collected whole blood in container 18 is passed through the leukocyte reduction filter 28 and flowed into transfer container 20, so that container 20 hold leukocyte reduced whole blood.

Container 20 is then centrifuged to separate the blood within container 20 into layers of its components. The heavier cellular components, such as red blood cells, settle to the bottom of the container 20 and the lighter, less dense components, such as plasma (with or without platelets), remain in the top layer. The components may then be separated by expressing the lighter components through tubings 30, 32 and into container 22. The heavier components, i.e., the concentrated red blood cells may be retained in the transfer container 20, to which a preservative/additive solution contained in container 24 may be flowed through tubings 34 and 30 after plasma has been expressed to container 22.

A volume of the leukocyte reduced, anticoagulated whole blood is retained in tubing 32, and a series of heat seals 42 are formed at spaced intervals along the length of the tubing segment to define discrete tube segments 44, each containing a sufficient volume of blood to permit, e.g., cross matching the donated blood component with the patient's blood prior to infusion.

To insure that the sample tested corresponds to the component in the container that is to be reinfused, the tubing 32 typically has had an alphanumeric identifier 46 printed thereon at regular intervals so that each tube segment 44 has the same alphanumeric identifier. The operator reads and records the alphanumeric identifier 46 and compares it to the alphanumeric identifier associated with the container holding the blood component to be reinfused. This introduces the possibility of human error in the reading and recording of the alphanumeric identifier 46 associated with the tubing segments 44, as well as in the comparison of the alphanumeric identifiers of the tube segment 44 and the container.

To reduce the possibility of such errors, and to provide for greater speed and efficiencies in record keeping, the tubing segments are each provided with a machine-readable identifier equivalent to the alphanumeric identifier, such as a barcode 48. The barcode 48 may be in place of or in addition to the alphanumeric identifier and automatically applied to the tubings of the processing set at the time of manufacture. A standard barcode prints black bars on white or shining background, which provide a good contrast to the black bars. While a standard bar code will work on a transparent tube, once the tube is filled (and in particular, filled with a red cell concentrate, RCC), black bars will have not enough contrast versus the background to be read with a bar code scanner. Thus, because the tubing segments have a degree of transparency and to achieve a good contrast of the bar code when the tube segment is filled, the bar code applied to the tubing segments is a negative or inverted barcode in which the black and white segments are reversed. While the transparent and natural color of the tubing provides a sufficient contrast to the printed white (negative) bars, the contrast improves significantly once the tubing is filled (or contaminated) with the designated content.

Thus, the automated scanning of the unique segment number using a barcode scanner is provided, while the alphanumeric information is maintained and errors in the manual entry of the segment number avoided. Further, the scanned information can be automatically added to the other records in the database of the blood collection center to insure completeness.

The present invention has been described in accordance with the preferred embodiments. Of course, it will be understood that the present invention is not limited to the processing set shown in the figures, and that processing sets having different container and tubing configurations are also within the scope of the present invention as defined in the appended claims.

## Claims

1. A blood donation system comprising:
a) a donor access device (12);
b) a first collection container (18) having an inlet port and an outlet port;
c) a first tubing (16) connecting the donor access device (12) to the inlet port of the first collection container (18);
d) a leukocyte reduction filter (30) having an inlet and an outlet;
e) a second collection container (20) having an inlet port and an outlet port;
f) a second tubing (28) connecting the outlet port of the first collection container (18) to the inlet of the leukocyte reduction filter (30);
g) a third tubing (32) connecting the outlet of the leukocyte reduction filter (30) to the inlet port of the second collection container (20);
h) wherein the system has a negative barcode (48) as machine-readable identification code associated therewith and the machine-readable identification code is printed on one of the tubings (16, 28, 32) at repeated intervals to define a plurality of discrete segments (44), each sized to contain a sample volume of biological fluid.

2. The blood donation system of claim 1 wherein the negative barcode as machine-readable identification code is printed on the third tubing segment (32).

3. The blood donation system of any one of claims 1-2 wherein the first collection container (18) is prefilled with a volume of anticoagulant.

4. The blood donation system of any one of claims 1-3 wherein an alphanumeric equivalent (46) of the machine-readable identification code is printed on each discrete segment (44) of the tubing in conjunction with negative barcode (48) as the machine-readable identification code.

5. A blood donation system comprising a plurality of containers (18, 20, 22, 24) interconnected by a plurality of tubing segments (16, 28, 32, 34, 36, 38), at least one tubing segment having a negative barcode (48) as machine-readable identification code associated therewith printed on the tubing segment at repeated intervals to define a plurality of discrete tubing sub-segments (44), each sized to contain a sample volume of biological fluid.

6. The blood donation system of claim 5 wherein an alphanumeric equivalent (46) of the machine-readable identification code is printed on each discrete (44) segment of the tubing in conjunction with the negative barcode (48) as the machine-readable identification code.

7. The blood donation system of any one of claims 5-6, wherein the plurality of discrete segments (44) are further defined by heat seals (42) formed at intervals along the tubing segment (32) connecting the outlet of the leukocyte reduction filter (30) to the inlet port of the second collection container (20).

8. The blood donation system of any one of claims 1-4, wherein the plurality of discrete segments (44) are further defined by heat seals (42) formed at intervals along the tubing (32) connecting the outlet of the leukocyte reduction filter (30) to the inlet port of the second collection container (20).

## Patentansprüche

1. Blutspendesystem, umfassend:
a) eine Spenderzugangsvorrichtung (12);
b) einen ersten Sammelbehälter (18) mit einem Einlassstutzen und einem Auslassstutzen;
c) einen ersten Schlauch (16), der die Spenderzugangsvorrichtung (12) mit dem Einlassstutzen des ersten Sammelbehälters (18) verbindet;
d) einen Leukozytenreduktionsfilter (30) mit einem Einlass und einem Auslass;
e) einen zweiten Sammelbehälter (20) mit einem Einlassstutzen und einem Auslassstutzen;
f) einen zweiten Schlauch (28), der den Auslassstutzen des ersten Sammelbehälters (18) mit dem Einlass des Leukozytenreduktionsfilters (30) verbindet;
g) einen dritten Schlauch (32), der den Auslass des Leukozytenreduktionsfilters (30) mit dem Einlassstutzen des zweiten Sammelbehälters (20) verbindet;
h) wobei das System einen Negativbarcode (48) als damit assoziierten maschinenlesbaren Identifikationscode aufweist, und der maschinenlesbare Identifikationscode in wiederholten Abständen auf einen der Schläuche (16, 28, 32) aufgedruckt ist, um eine Vielzahl von diskreten Segmenten (44) zu definieren, die jeweils dazu bemessen sind, einen Probenvolumen an biologischen Fluid zu enthalten.

2. Blutspendesystem nach Anspruch 1, wobei der Negativbarcode als maschinenlesbarer Identifikationscode auf das dritte Schlauchsegment (32) aufgedruckt ist.

3. Blutspendesystem nach einem der Ansprüche 1-2, wobei der erste Sammelbehälter (18) mit einem Volumen an Gerinnungshemmer vorgefüllt ist.

4. Blutspendesystem nach einem der Ansprüche 1-3, wobei auf jedes diskrete Segment (44) des Schlauchs eine alphanumerische Entsprechung (46) des maschinenlesbaren Identifikationscodes in Verbindung mit dem Negativbarcode (48) als der maschinenlesbare Identifikationscode aufgedruckt ist.

5. Blutspendesystem, umfassend eine Vielzahl von Behältern (18, 20, 22, 24), die durch eine Vielzahl von Schlauchsegmenten (16, 28, 32, 34, 36, 38) miteinander verbunden sind, wobei mindestens ein Schlauchsegment einen Negativbarcode (48) als damit assoziierten maschinenlesbaren Identifikationscode aufweist, der in wiederholten Abständen auf das Schlauchsegment aufgedruckt ist, um eine Vielzahl von diskreten Schlauchteilsegmenten (44) zu definieren, die jeweils dazu bemessen sind, ein Probenvolumen an biologischem Fluid zu enthalten.

6. Blutspendesystem nach einem Anspruch 5, wobei auf jedes diskrete Segment (44) des Schlauchs eine alphanumerische Entsprechung (46) des maschinenlesbaren Identifikationscodes in Verbindung mit dem Negativbarcode (48) als der maschinenlesbare Identifikationscode aufgedruckt ist.

7. Blutspendesystem nach einem der Ansprüche 5-6, wobei die Vielzahl von diskreten Segmenten (44) weiter durch Heißsiegel (42) definiert sind, die in Abständen entlang des den Auslass des Leukozytenreduktionsfilters (30) mit dem Einlassstutzen des zweiten Sammelbehälters (20) verbindenden Schlauchsegments (32) gebildet sind.

8. Blutspendesystem nach einem der Ansprüche 1-4, wobei die Vielzahl von diskreten Segmenten (44) weiter durch Heißsiegel (42) definiert sind, die in Abständen entlang des den Auslass des Leukozytenreduktionsfilters (30) mit dem Einlassstutzen des zweiten Sammelbehälters (20) verbindenden Schlauchs (32) gebildet sind.

## Revendications

1. Système de don de sang comprenant :
a) un dispositif d'accès au donneur (12) ;
b) un premier récipient de collecte (18) présentant un orifice d'entrée et un orifice de sortie ;
c) une première tubulure (16) reliant le dispositif d'accès au donneur (12) à l'orifice d'entrée du premier récipient de collecte (18) ;
d) un filtre de réduction de leucocytes (30) présentant une entrée et une sortie ;
e) un deuxième récipient de collecte (20) présentant un orifice d'entrée et un orifice de sortie ;
f) une deuxième tubulure (28) reliant l'orifice de sortie du premier récipient de collecte (18) à l'entrée du filtre de réduction de leucocytes (30) ;
g) une troisième tubulure (32) reliant la sortie du filtre de réduction de leucocytes (30) à l'orifice d'entrée du deuxième récipient de collecte (20) ;
h) dans lequel le système présente un code à barres négatif (48) en tant que code d'identification lisible par machine associé à celui-ci et le code d'identification lisible par machine est imprimé sur une des tubulures (16, 28, 32) à des intervalles répétés pour définir une pluralité de segments distincts (44), chaque segment étant dimensionné pour contenir un volume d'échantillon de fluide biologique.

2. Système de don de sang selon la revendication 1, dans lequel le code à barres négatif en tant que code d'identification lisible par machine est imprimé sur le troisième segment de tubulure (32).

3. Système de don de sang selon l'une quelconque des revendications 1-2, dans lequel le premier récipient de collecte (18) est prérempli avec un volume d'anticoagulant.

4. Système de don de sang selon l'une quelconque des revendications 1-3, dans lequel un équivalent alphanumérique (46) du code d'identification lisible par machine est imprimé sur chaque segment distinct (44) de la tubulure en conjonction avec le code à barres négatif (48) en tant que code d'identification lisible par machine.

5. Système de don de sang comprenant une pluralité de récipients (18, 20, 22, 24) interconnectés par une pluralité de segments de tubulure (16, 28, 32, 34, 36, 38) sur au moins un segment de tubulure présentant un code à barres négatif (48) en tant que code d'identification lisible par machine associé à celui-ci imprimé sur le segment de tubulure à des intervalles répétés pour définir une pluralité de sous-segments de tubulure distincts (44), chacun étant dimensionné pour contenir un volume d'échantillon de fluide biologique.

6. Système de don de sang selon la revendication 5, dans lequel un équivalent alphanumérique (46) du code d'identification lisible par machine est imprimé sur chaque segment distinct (44) de la tubulure en conjonction avec le code à barres négatif (48) en tant que le code d'identification lisible par machine.

7. Système de don de sang selon l'une quelconque des revendications 5-6, dans lequel la pluralité de segments distincts (44) sont en outre définis par des joints thermoscellables (42) formés à des intervalles le long du segment de tubulure (32) reliant la sortie du filtre de réduction de leucocytes (30) à l'orifice d'entrée du deuxième récipient de collecte (20).

8. Système de don de sang selon l'une quelconque des revendications 1-4, dans lequel la pluralité de segments distincts (44) sont en outre définis par des joints thermoscellables (42) formés à des intervalles le long de la tubulure (32) reliant la sortie du filtre de réduction de leucocytes (30) à l'orifice d'entrée du deuxième récipient de collecte (20).
